(19) Europäisches Patentamt / European Patent Office / Office européen des brevets

(11) **EP 1 933 591 B1**

(12) **EUROPÄISCHE PATENTSCHRIFT**

(45) Veröffentlichungstag und Bekanntmachung des Hinweises auf die Patenterteilung:
**16.01.2013 Patentblatt 2013/03**

(51) Int Cl.:
***H04R 25/00*** *(2006.01)*

(21) Anmeldenummer: **07022519.8**

(22) Anmeldetag: **20.11.2007**

(54) **Verfahren zur Bestimmung der individuellen Hörfähigkeit**

Method for determining individual hearing ability

Procédé de détermination de la capacité auditive individuelle

(84) Benannte Vertragsstaaten:
**AT BE BG CH CY CZ DE DK EE ES FI FR GB GR HU IE IS IT LI LT LU LV MC MT NL PL PT RO SE SI SK TR**

(30) Priorität: **12.12.2006 DE 102006058522**

(43) Veröffentlichungstag der Anmeldung:
**18.06.2008 Patentblatt 2008/25**

(73) Patentinhaber: **GEERS Hörakustik AG & Co. KG
44227 Dortmund (DE)**

(72) Erfinder:
• **Seidler, Hannes, Dr.-Ing.
01097 Dresden (DE)**

• **Haubold, Jörg, Dr.-Ing.
44287 Dortmund (DE)**

(74) Vertreter: **Manitz, Finsterwald & Partner GbR
Postfach 31 02 20
80102 München (DE)**

(56) Entgegenhaltungen:
EP-A- 0 674 462          EP-A- 1 239 269
US-A1- 2006 093 997

• JONGE DE R: "HEARING AID SELECTION USING A MICROCOMPUTER" HEARING INSTRUMENTS, HARCOURT BRACE JOVANOVICH PUBL. DULUTH, MINNESOTA, US, Bd. 38, Nr. 5, Mai 1987 (1987-05), Seiten 126-130, XP000796160 ISSN: 0092-4466

**Beschreibung**

Die vorliegende Erfindung bezieht sich auf das Gebiet der Hörgeräteanpassung. Insbesondere bezieht sich die vorliegende Erfindung auf die Bestimmung der individuellen Hörfähigkeit, die zur Anpassung von Hörgeräten erforderlich ist.

Als Hörverlust wird die Verringerung der Hörfähigkeit eines Menschen im Vergleich zu einem gesunden Hörorgan bezeichnet. Er kann durch unterschiedliche Ursachen entstehen, z.B. durch eine Lärmexposition, Verletzungen des Ohres, erbliche Erkrankungen oder altersbedingte Hörverluste. Betroffen sein können anatomische Bereiche wie das Außenohr, das Mittelohr, das Innenohr, der Hörnerv oder das Gehirn.

Die individuelle Hörfähigkeit entspricht also der ausgehend von der Hörfähigkeit eines Normalhörenden unter Berücksichtigung des individuellen Hörverlustes verbliebenen Hörfähigkeit eines Menschen.

Bei den beispielsweise aus der EP-A-1 239 269 bekannten Methoden wird die Hörfähigkeit mit audiometrischen Methoden bestimmt. In der täglichen HNO-Praxis wird vorrangig die Tonaudiometrie zur Bestimmung der Hörschwelle verwendet. Die Hörschwelle bezeichnet dabei die untere Wahrnehmungsgrenze des Menschen für Schalle, an der Schallsignale gerade noch wahrgenommen werden. Die Signale werden mit einer vorgegebenen Frequenz in der Regel über einen Kopfhörer dargeboten. Bei Steigerung des Schallpegels wird geprüft, bei welchem Schallpegel der Proband den Ton/das Geräusch gerade noch wahrnimmt. Die dazu notwendigen technischen Systeme werden als Audiometer bezeichnet.

Die Ergebnisse der audiometrischen Messung werden in Dezibel Hearing Level (dBHL) angegeben und frequenzabhängig in ein Audiogramm eingetragen.

Neben dem beschriebenen Verfahren zur subjektiven Audiometrie, wird bei der objektiven Audiometrie eine Reaktion der Nerven und Hirnregionen auf Schallreizungen bestimmt, um die Hörfähigkeit zu beschreiben.

Die Bestimmung und Klassifizierung einer Hörfähigkeit bildet die Grundlage einer Hörgeräteversorgung. Die ermittelte Hörfähigkeit wird sowohl zur Verordnung von Hörgeräten als auch zur Einstellung bzw. Nacheinstellung von Hörgeräten angewendet werden.

Die bekannte Messung der Hörfähigkeit erfordert den Einsatz von Audiometern, was zusätzlich zu den Investitionen laufende Wartungskosten verursacht, da die Messtechnik den Anforderungen des Medizinproduktegesetzes unterliegt. Standardmessungen eines Audiogramms erfolgen in der Regel in 5 dB-Stufen. Diese Messabstufung stellt ein Maß an Ungenauigkeit in der Bestimmung der Hörfähigkeit dar.

Bekannt ist, dass die Werte eines Hörverlustes nicht vollständig in die Bestimmung der Einstellung einer Hörtechnik berücksichtigt werden. Im Allgemeinen wird maximal die Hälfte eines Hörverlustes als Verstärkungswert ermittelt, so dass sich mögliche Messfehler eines Hörverlustes ebenfalls nur mit maximal 50% auf eine Hörtechnikeinstellung auswirken.

Aufgabe der vorliegenden Erfindung ist es, ein Verfahren zur Bestimmung der individuellen Hörfähigkeit anzugeben, das eine Einstellung der Hörgeräte in Abhängigkeit der Hörfähigkeit ermöglicht und das kostengünstig und einfach durchzuführen ist.

Diese Aufgabe wird mit einem Verfahren zur Bestimmung der individuellen Hörfähigkeit mit den Merkmalen des Anspruchs 1 gelöst. Unteransprüche sind auf bevorzugte Ausgestaltungen gerichtet. Anspruch 14 ist auf ein Verfahren zur Hörgeräteanpassung unter Einsatz eines erfindungsgemäßen Verfahrens zur Bestimmung der individuellen Hörfähigkeit gerichtet.

Das erfindungsgemäße Verfahren zur Bestimmung der individuellen Hörfähigkeit bestimmt zunächst den statistischen frequenzabhängigen Altershörverlust. Die so bestimmten Werte werden als Ausgangspunkt für die Bestimmung der individuellen Hörfähigkeit des Probanden verwendet. Der Proband wird zusätzlich einer strukturierten Befragung unterzogen, aus der weitere Informationen zur Bestimmung der individuellen Hörfähigkeit gezogen werden. Unter Berücksichtigung der Ergebnisse der strukturierten Befragung werden die Werte für die Abschätzung des individuellen Hörverlustes energetisch korrigiert, um korrigierte Werte zu erhalten. Dabei kann zusätzlich wie bei der Durchführung einer messtechnischen Audiometrie mit einer 5 dB-Abstufung gerundet werden.

Die korrigierten Werte können für die Einstellung von Hörgeräteparametern verwendet werden.

Die frequenzabhängigen Werte für die Abschätzung des individuellen Hörverlustes sind abhängig vom Alter des Probanden. Bei der Abschätzung des Altershörverlustes aus statistischen Werten wird außerdem in der Regel das Geschlecht des Probanden berücksichtigt.

Die strukturierte Befragung kann Fragen unter anderem z.B. zu den folgenden Themen enthalten: Erwartungen des Kunden, bisherige Hörgeräteversorgung, bevorzugte Gesprächsseite, wichtige Lebenssituationen, Schwerpunkte in den Hörumgebungsbereiche, kritische Hörsituationen, Wahrnehmung der eigenen Stimme, Telefonbenutzung, soziales Umfeld, medizinische Aspekte, motorische Fähigkeiten oder ähnliches. Die Antworten der strukturierten Befragung hinsichtlich dieser oder ähnlicher Themen können bei der Bestimmung der individuellen Hörfähigkeit des Probanden eingesetzt werden.

Durch die Verwertung der Ergebnisse der strukturierten Befragung ausgehend von dem statistischen frequenzabhängigen Altershörverlust ist es möglich, die individuelle Hörfähigkeit des Probanden zu bestimmen, so dass auf die Verwendung eines kostspieligen und wartungsintensiven Audiometers verzichtet werden kann.

Der statistische frequenzabhängige Altershörverlust kann aus entsprechenden Diagrammen abgelesen werden. Insbesondere bei teilweiser oder vollständiger Automatisierung des Verfahrens, kann es sich als vorteilhaft erweisen, wenn mit Hilfe einer Formel der altersabhängige Hörverlust aus den Daten des Probanden berechnet werden kann. Vorteilhafterweise wird dementsprechend zur Bestimmung des altersabhängigen Hörverlustes eine Berechnung gemäß der ISO-Norm 1999 wie folgt verwendet:

$$HV(f) = 1,57 \cdot a(f) \cdot (Y-18)^2 + 1,28 \cdot b(f)$$

wobei a und b Funktionen der Frequenz f und Y das Alter des Probanden ist. Diese Formel gestattet eine einfache Bestimmung der frequenzabhängigen Startwerte für die Abschätzung des Hörverlustes. Die einfließenden Parameter a und b können z.B. aus einer geschlechtsabhängigen Tabelle für unterschiedliche Frequenzen abgelesen oder für eine automatische Berechnung in Speichereinrichtungen hinterlegt werden. Vorzugsweise erfolgt ein Runden in einer 5 dB-Abstufung.

Aus der strukturierten Befragung können unterschiedliche Informationen extrahiert werden, die klassifiziert und zur energetischen Korrektur der Werte für die Abschätzung der individuellen Hörfähigkeit des Probanden verwendet werden. Insbesondere kann aus der strukturierten Befragung auf einen Hörschadenstyp des Probanden geschlossen werden. So lassen sich z.B. aus den Antworten des Probanden auf Vorerkrankungen oder besondere Belastungssituationen schließen. Diese Antwortkategorien erlauben eine Einordnung des individuellen Hörschadens. Für die einzelnen Kategorien können z.B. in Tabellen oder Speichereinrichtungen Korrekturtabellen abgelegt sein, die frequenzabhängig eine der Kategorie entsprechende Korrektur der individuellen Hörfähigkeit angeben. Liegen mehrere Hörschadenstypen vor, werden diese unabhängig voneinander berücksichtigt.

Bei einer weiteren Ausgestaltung kann in die Korrektur des altersbedingten Hörverlustes die Sprachverständlichkeit einfließen. Insbesondere aus Fragen der strukturierten Befragung zum Sprachverstehen im Störgeräusch wird eine Korrektur für die Abschätzung des individuellen Hörverlustes bestimmt. Dazu können bei einer bevorzugten Ausgestaltung mehrere, bevorzugt wenigstens drei Situationen für Sprachverstehen im Störgeräusch verwendet und gemittelt werden. Der Proband gibt sein subjektives Empfinden der Sprachverständlichkeit in mehreren Stufen, bevorzugt fünf Stufen an. Zur optimalen Berücksichtigung der Sprachverständlichkeit zur Ermittlung der entsprechenden Korrekturwerte für die Abschätzung des individuellen Hörverlustes wird die Sprachverständlichkeitsstufe mit ausgewählten frequenzabhängigen Signalpegeln multipliziert. Bei einer vorteilhaften Ausgestaltung wird mit Hilfe einer entsprechend ausgewählten frequenzabhängigen Wichtung der sprachrelevante Frequenzbereich stärker gewichtet. Vorzugsweise erfolgt abschließend ein Runden in einer 5 dB-Abstufung.

Bei einer weiteren Ausgestaltung des erfindungsgemäßen Verfahrens zur Bestimmung der Hörfähigkeit wird die Unbehaglichkeitsschwelle des Probanden berücksichtigt. Auf diese Weise kann bei der Hörgeräteeinstellung verhindert werden, dass Einstellungen gewählt werden, die zu zu großen Lautstärkeübertragungen durch ein Hörgerät führen. Aus der strukturierten Befragung kann auf individuelle Abweichungen der Unbehaglichkeitsschwelle des potentiellen Hörgeräteanwenders von einem Normalwert geschlossen werden. Entsprechend der dargebotenen Antwortkategorien kann dann die abgeschätzte Unbehaglichkeitsschwelle erhöht bzw. erniedrigt werden.

Die strukturierte Befragung kann auch derart erweitert werden, dass Informationen über die seitenspezifische Wahrnehmung des Probanden erhalten werden, die dann ebenfalls bei der energetischen Korrektur der Abschätzung des individuellen Hörverlustes berücksichtigt werden können. Vorzugsweise erfolgt abschließend ein Runden in einer 5 dB-Abstufung.

Besonders vorteilhaft ist es, wenn der Proband z.B. als abschließenden Schritt einer Auralisierung unterzogen wird. Dabei werden dem Probanden mit einem handelsüblichen akustischen Wiedergabesystem definierte Schalleigenschaften vorgespielt. Der Proband hat die Möglichkeit mit den bekannten Einstellmöglichkeiten die Lautstärke, die Balance und/oder die Klangeinstellung zu ändern, um sein subjektives Empfinden in die Bestimmung der individuellen Hörfähigkeit mit einfließen zu lassen.

Einzelne der geschilderten Ausgestaltungen, bevorzugt alle geschilderten Ausgestaltungen, können vorteilhafterweise miteinander kombiniert werden, um die Bestimmung der individuellen Hörfähigkeit zu optimieren.

Besonders einfach ist die Durchführung des erfindungsgemäßen Verfahrens, wenn die Abschätzung des Hörverlustes in dBHL angegeben wird und die Korrekturwerte ebenfalls in Dezibel bestimmt werden. Die energetische Addition der aus dem Altershörverlust bestimmten Startwerte für die Abschätzung des individuellen Hörverlustes und den Korrekturwerten, die sich aus der strukturierten Befragung ergeben, liefert dann vorzugsweise nach abschließendem Runden in einer 5 dB-Abstufung auf einfache Weise den individuellen Hörverlust, der bei der Einstellung der Hörgeräte verwendet werden kann.

Eine "energetische Addition" erfolgt dabei z.B. analog zu dem allgemein bekannten, physikalischen Gesetz der Schallpegeladdition, das zur Erläuterung angegeben wird:

$$L_{ges} = 10 \cdot \lg \sum_{i=1}^{n} 10^{0,1 L_i}$$

wobei Li der Schalldruckpegel der einzelnen Schallsignale und $L_{ges}$ der resultierende Gesamtschalldruckpegel sind ($\lg$ steht für den Logarithmus zur Basis 10). Bei dem erfindungsgemäßen Verfahren und seinen Ausgestaltungen kann die energetische Addition nach derselben Vorschrift vorgenommen werden, wobei in der angegebenen Formel anstelle der Schalldruckpegel Li der statische frequenzabhängige Altershörverlust und die gegebenenfalls ermittelten Korrekturwerte eingesetzt werden, um im Ergebnis anstelle des (bei der Schallpegeladdition erhaltenen) Gesamtschalldruckpegels $L_{ges}$ den individuellen Hörverlust zu erhalten.

Das erfindungsgemäße Verfahren, das die Ergebnisse einer strukturierten Befragung des Probanden ausgehend von dem frequenzabhängigen Altershörverlust zur Bestimmung der individuellen Hörfähigkeit verwendet, ermöglicht den Verzicht auf sonstiges teures und wartungsintensives E-quipment.

Das erfindungsgemäße Verfahren wird im Folgenden anhand einer typischen Verfahrensführung im Detail erläutert.

Fig. 1   zeigt den Hörverlust in dBHL (Dezibel Hearing Level) in einer 5 dB-Abstufung in Abhängigkeit der Frequenz für verschiedene Altersgruppen für männliche Personen, und

Fig. 2   zeigt den Hörverlust in dBHL (Dezibel Hearing Level) in einer 5 dB-Abstufung in Abhängigkeit der Frequenz für verschiedene Altersgruppen für weibliche Personen.

Aus dem Alter und dem Geschlecht des Probanden, zum Beispiel eines Hörgeschädigten, wird auf den frequenzabhängigen Altershörverlust HV(f) geschlossen. Dazu wird bei der beschriebenen Ausführungsform eine Gleichung wie folgt verwendet:

$$HV(f) = 1{,}57 \cdot a(f) \cdot (Y-18)^2 + 1{,}28 \cdot b(f)$$

Dabei sind a und b Funktionen der Frequenz f. Y ist das Alter des Probanden in Jahren. Diese Formel entspricht dem Altershörverlust wie er nach ISO 1999 bestimmt werden kann.

Die Werte für die verwendeten Parameter können z.B. gemäß Tabelle 1 abgelesen werden.

TABELLE 1:

| Frequenz in kHz | 0,125 | 0,25 | 0,5 | 1 | 1,5 | 2 | 3 | 4 | 6 | 8 |
|---|---|---|---|---|---|---|---|---|---|---|
| a männlich | 0,0030 | 0,0030 | 0,0035 | 0,0040 | 0,0055 | 0,0070 | 0,0115 | 0,0160 | 0,0180 | 0,0220 |
| a weiblich | 0,0030 | 0,0030 | 0,0035 | 0,0040 | 0,0050 | 0,0060 | 0,0075 | 0,0090 | 0,0120 | 0,0150 |
| b männlich | 7,23 | 6,67 | 6,12 | 6,12 | 6,67 | 7,23 | 7,78 | 8,34 | 9,45 | 10,56 |
| b weiblich | 6,67 | 6,12 | 6,12 | 6,12 | 6,67 | 6,67 | 7,23 | 7,78 | 8,90 | 10,56 |

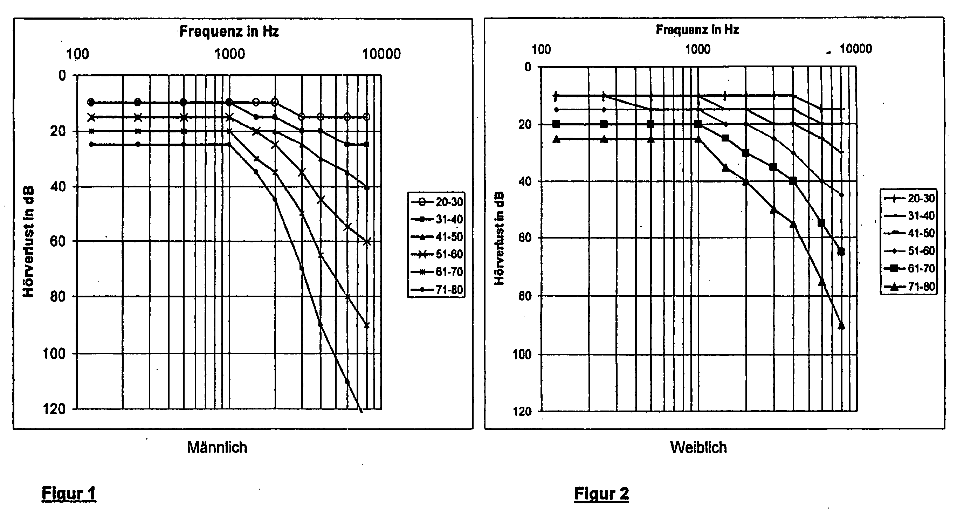

Aus der Formel kann eine grafische Übersicht berechnet werden, die bei einer alternativen Ausgestaltung des Verfahrens direkt zum Ablesen des frequenzabhängigen Hörverlustes für unterschiedliche Altersgruppen dienen kann. Die entsprechenden grafischen Übersichten sind in Fig. 1 und 2 gezeigt. In den Grafiken sind die verschiedenen Kurven der jeweils rechts von der jeweiligen Grafik angegebenen Altersgruppen geschlechtsspezifisch in einer 5 dB-Abstufung angegeben.

Der so bestimmte Hörverlust in dBHL wird als Startwert für die Bestimmung der individuellen Hörfähigkeit verwendet.

Der Proband wird einer strukturierten Befragung unterzogen, die z.B. Rückschlüsse auf den Hörschadenstyp und die Sprachverständlichkeit geben.

Insbesondere werden z.B. die Erwartungen des Probanden, die bisherige Hörgeräteversorgung, die bevorzugte Gesprächsseite, wichtige Lebenssituationen, die Schwerpunkte in dem Hörumgebungsbereich, kritische Hörsituationen, Wahrnehmung der eigenen Stimme, Telefonbenutzung, soziales Umfeld, medizinische Aspekte und motorische Fähigkeiten abgefragt. Aus den Antworten auf einen entsprechenden Fragenkatalog wird auf den Hörschadenstyp geschlossen. Dies wird im Folgenden anhand von ausgewählten Beispielen erläutert.

Ergeben die Antworten der strukturierten Befragung z.B., dass der Proband ein entzündliches Mittelohr hat, an Viruserkrankungen leidet oder gelitten hat, bestimmte Medikamente einnimmt oder einer starken Lärmbelastung ausgesetzt ist bzw. war, so lässt sich auf den Hörschadenstyp einer basocochleären Innenohrschwerhörigkeit schließen.

Hat der Proband einen Hörsturz erlitten, wird auf eine apikocochleäre Innenohrschwerhörigkeit geschlossen.

Ergeben insbesondere Fragen zu den medizinischen Aspekten, dass der Proband an Morbus Menière leidet oder gelitten hat, wird sein Hörschaden als pancochleäre Innenohrschwerhörigkeit klassifiziert.

Ergibt die strukturierte Befragung, dass der Proband kritischen Hörsituationen, insbesondere hohen Schallbelastungen, ausgesetzt war und insofern ein Schalltrauma erlitten hat, so wird sein Hörschadenstyp als $c^5$-Senke klassifiziert.

In ähnlicher Weise lassen sich die Ergebnisse der Befragung zur Klassifizierung in weitere Hörschadenstypen einordnen.

Verschiedene Hörschadenstypen sind beispielhaft in Tabelle 2 angegeben (nach Dieroff H.-G.: Lärmschwerhörigkeit, Gustav Fischer Verlag, Jena, Stuttgart 1994).

TABELLE 2:

| *Hörschadenstyp* | **Differenzierung** | **Beschreibung** |
|---|---|---|
| *Mittelohrschwerhörigkeit* | Versteifungstyp | Luftleitung bei tiefen Frequenzen 10-60 dB, beginnende Otosklerose, Schädelunfälle |
| | Kombinationstyp | Luftleitung breitbandig bis 60 dB maximal, Otosklerose |
| *Innenohrschwerhörigkeit* | Basocochleäre Innenohrschwerhörigkeit (Hochtonschwerhörigkeit) | schwacher bis steiler Abfall in den Höhen, Alter, entzündliche Mittelohr, Intoxikation, Viruskrankheiten, Medikamente, Lärm, Knall, Schädeltrauma |
| | Mediocochleäre Innenohrschwerhörigkeit (Mitteltonschwerhörigkeit) | Abfall nur in mittleren Bereichen (Sprache) - sehr selten, Hörnerv |
| | Apikocochleäre Innenohrschwerhörigkeit (Tieftonschwerhörigkeit) | Abfall bei tiefen Frequenzen - selten, beginnende Morbus Meniere, Hörsturz |
| | Pancochleäre Innenohrschwerhörigkeit (pantonale Schwerhörigkeit) | breitbandig mehr oder weniger geschädigt, Morbus Meniere |
| *Hörsenken* | Hochtonbereich | $c^5$-Senke (4000 Hz), Schalltrauma, Schädeltrauma, Intoxikation |
| | Mitteltonbereich | äußerst selten |
| *Hörsymmetrie* | | Erbkrankheiten, Lärm, Medikamente, Infektionen; Contra: Morbus Meniere, Explosion, Knalltrauma |
| *Recruitment* | | Knall-, Lärmtrauma; Contra: Altersschwerhörigkeit |

Entsprechend der Klassifizierung des Hörschadens des Probanden werden frequenzabhängige Korrekturwerte für die Abschätzung des individuellen Hörverlustes bestimmt. Dazu können Tabellen eingesetzt oder für eine automatische Berechnung Speichereinrichtungen verwendet werden, wie sie beispielhaft für einige Hörschadenstypen im Folgenden angegeben werden.

TABELLE 3: Empfohlene Korrekturwerte bei einer basocochleären Innenohrschwerhörigkeit:

| Frequenz in kHz | 0,25 | 0,5 | 1 | 2 | 4 | 8 |
|---|---|---|---|---|---|---|
| Hörverlustkorrektur in dBHL | 0 | 0 | 0 | 50 | 70 | 90 |

TABELLE 4: Empfohlene Korrekturwerte bei einer apikocochleären Innenohrschwerhörigkeit:

| Frequenz in kHz | 0,25 | 0,5 | 1 | 2 | 4 | 8 |
|---|---|---|---|---|---|---|
| Hörverlustkorrektur in dBHL | 40 | 40 | 30 | 15 | 0 | 0 |

TABELLE 5: Empfohlene Korrekturwerte bei einer pancochleären Innenohrschwerhörigkeit:

| Frequenz in kHz | 0,25 | 0,5 | 1 | 2 | 4 | 8 |
|---|---|---|---|---|---|---|
| Hörverlustkorrektur in dBHL | 50 | 50 | 50 | 50 | 50 | 50 |

TABELLE 6: Empfohlene Korrekturwerte bei einer $c^5$-Senke:

| Frequenz in kHz | 0,25 | 0,5 | 1 | 2 | 4 | 8 |
|---|---|---|---|---|---|---|
| Hörverlustkorrektur in dBHL | 0 | 0 | 0 | 10 | 40 | 20 |

Die so bestimmten Korrekturwerte in dBHL werden zu dem altersbedingten Hörverlust in dBHL energetisch addiert, vorzugsweise abschließend in einer 5 dB-Abstufung gerundet, und bilden so eine erste Korrektur des altersbedingten Hörverlustes. Die energetische Addition erfolgt nach der oben angegebenen Vorschrift analog zu einer Schallpegeladdition.

Insbesondere mit Fragen zum Sprachverstehen in unterschiedlichen akustischen Umgebungen kann die wahrgenommene Sprachverständlichkeitsstufe bestimmt werden. Der Proband klassifiziert das subjektive Empfinden der Sprachverständlichkeit in z.B. fünf Stufen (1 = deutlich, 2 = gut, 3 = ausreichend, 4 = kaum, 5 = gar nicht). Die Sprachverständlichkeitsstufe wird mindestens für drei unterschiedliche Situationen ermittelt. Die Ergebnisse werden gemittelt, um eine gemittelte Sprachverständlichkeitsstufe weiter verwenden zu können. Die gemittelte Sprachverständlichkeitsstufe wird mit einem frequenzabhängigen Signalpegel multipliziert, wobei es empfehlenswert ist, Signalpegel entsprechend einer real vorliegenden Situation einzusetzen. Mit Hilfe einer frequenzabhängigen Wichtung werden die mittleren Frequenzen im sprachrelevanten Bereich bevorzugt. Tabelle 7 gibt frequenzabhängige Signalpegel und Wichtungen an, die bei einer Berücksichtigung der Sprachverständlichkeit eingesetzt werden können.

TABELLE 7:

| Frequenz in kHz | 0,25 | 0,5 | 1 | 2 | 4 | 8 |
|---|---|---|---|---|---|---|
| Signalpegel in dB | 67 | 67 | 61 | 58 | 50 | 49 |
| Wichtung | 0,135 | 0,135 | 0,153 | 0,145 | 0,125 | 0,073 |

In einem weiteren Schritt wird eine mögliche Seitendifferenz des Hörens berücksichtigt. Aus den Informationen des Fragenkatalogs zum linken und rechten Hören, so wie z.B. der Ohrseite beim Telefonieren, wird eine Bewertung zur seitenspezifischen Wahrnehmung des Probanden erfasst. Die gemäß dem obigen Verfahren bestimmte Hörfähigkeit wird als Hörfähigkeit für das bessere Ohr verwendet, da dieses das allgemeine Hörvermögen im Wesentlichen bestimmt. Für die schlechtere Ohrseite werden Korrekturwerte für die Hörfähigkeit berücksichtigt, die z.B. der Tabelle 8 zu entnehmen sind.

TABELLE 8:

| Rechts - links - Bewertung | Zuschlag |
|---|---|
| "normal" und "schlecht" | +10 dBHL |
| "schlecht" und "fast nichts" | +20 dBHL |

(fortgesetzt)

| Rechts - links - Bewertung | Zuschlag |
|---|---|
| "normal" und "fast nichts" | +40 dBHL |

Die wie geschildert beschriebenen Korrekturwerte in dBHL für den Hörschadenstyp, die Sprachverständlichkeit und die seitendifferente Hörfähigkeit werden zu der ersten Abschätzung des individuellen Hörverlustes energetisch addiert und vorzugsweise abschließend in einer 5 dB-Abstufung gerundet, um einen korrigierten Hörverlust zu erhalten.

Bei der Bestimmung des Korrekturwertes für den altersbedingten Hörverlust zur Bestimmung der individuellen Hörfähigkeit wird zusätzlich die Unbehaglichkeitsschwelle berücksichtigt. Zunächst wird eine mittlere Unbehaglichkeitsschwelle potentieller Hörgeräteanwender zugrunde gelegt, wie z.B. die aus Tabelle 9 entnehmbaren Werte für eine frequenzabhängige Unbehaglichkeitsschwelle (UCL).

TABELLE 9:

| Frequenz in kHz | 0,5 | 1 | 2 | 4 |
|---|---|---|---|---|
| UCL in dBHL | 100 | 100 | 103 | 105 |

Aus der strukturierten Befragung kann auf individuelle Abweichungen der Unbehaglichkeitsschwelle des potentiellen Hörgeräteanwenders geschlossen werden. Entsprechend der möglichen Fragestellungen und Antwortmöglichkeiten, die beispielhaft in Tabelle 10 angegeben sind, kann dann die Unbehaglichkeitsschwelle erhöht bzw. erniedrigt werden.

TABELLE 10:

| schmerzhaft laut | -5 dBHL |
|---|---|
| laut ≡ keine Angaben | 0 dBHL |
| mittel | +5 dBHL |
| leise | +10 dBHL |
| gar nicht | + 15 dBHL |

Die individuelle Korrektur der Unbehaglichkeitsschwelle wird frequenzunabhängig vorgenommen.

Beispiel für eine Bestimmung eines Hörverlustes gemäß einer Ausgestaltung der Erfindung:

Ein Beispiel soll die Verwendung der geschilderten Ausgestaltungen erläutern. Die energetische Addition des altersbedingten Hörverlustes und der gegebenenfalls bestimmten Korrekturwerte erfolgt wiederum nach der oben angegebenen Vorschrift analog zu einer Schallpegeladdition.

Betrachtet wird ein männlicher Proband mit 65 Jahren. Aus Tabelle 1 und der Formel für den altersbedingten Hörverlust (oder durch Ablesen aus Fig. 1) ergibt sich der altersbedingte Hörverlust z.B. zu 21,7 dBHL bei der Frequenz 1 kHz. In einer 5 dB-Abstufung gerundet resultiert ein altersbedingter Hörverlust von 20 dBHL.

Die Antworten auf den Fragenkatalog ergeben, dass der Proband oft an einem entzündeten Mittelohr leidet. Es wird daher auf eine basocochleäre Innenohrschwerhörigkeit geschlossen. Aus Tabelle 3 wird der entsprechende Korrekturwert für die einzelnen Frequenzen bestimmt. Bei 1 kHz ist der Korrekturwert 0 dBHL einzusetzen.

Außerdem hat der Proband jedoch einen Hörsturz erlitten. Zusätzlich liegt also eine apikocochleäre Innenohrschwerhörigkeit vor. Bei einer Frequenz von 1 kHz muss daher der Hörverlust um den Wert 30 dBHL energetisch erhöht werden (Tabelle 4).

Andere Hörschadenstypen seien bei diesem Probanden nicht feststellbar.

Der Proband wird nach unterschiedlichen Situationen für das Sprachverstehen im Störgeräusch befragt. Er bewertet die Verständlichkeit der Sprache gemäß den oben angegebenen Sprachverständlichkeitsstufen. Bei einer ergibt sich beispielsweise die Sprachverständlichkeitsstufe als "gut", bei einer zweiten als "ausreichend" und bei einer dritten als "kaum". Die so ermittelten Sprachverständlichkeitsstufen (gut = 2, ausreichend = 3, kaum = 4) werden gemittelt, so dass sich eine gemittelte Sprachverständlichkeitsstufe von 3 ergibt. Um den Hörverlust bei 1 kHz entsprechend zu korrigieren, wird die Sprachverständlichkeitsstufe gemäß Tabelle 7 bei 1 kHz mit einem Signalpegel von 61 dB multipliziert und mit 0,153 gewichtet. Der Hörverlust für die Sprache ergibt sich also als 3 x 61 x 0,153 dBHL = 28 dBHL.

Der altersbedingte Hörverlust bei 1 kHz von ca. 22 dB wird also durch eine energetische Addition von 30 dB für den ermittelten Hörschadenstyp und 28 dBHL für den Sprachhörverlust vergrößert. Es ergibt sich ein in einer 5 dB-Abstufung gerundeter, individueller Hörverlust bei 1 kHz von 30 dBHL.

In einem weiteren Schritt kann die Seitendifferenz des Hörens berücksichtigt werden. Als Beispiel ergibt sich aus der Befragung des Probanden, z.B. dass er rechts normal und links schlecht hört. Die bisher ermittelte Hörverlustschätzung wird dann für das rechte Ohr angenommen. Das linke Ohr wird gemäß Tabelle 8 um 10 dBHL energetisch korrigiert, also weiterhin in einer 5 dB-Abstufung gerundet mit 30 dBHL angenommen.

Für den Probanden wird dann die frequenzabhängige Unbehaglichkeitsschwelle bestimmt. Aus den Angaben des Probanden kann eine Korrektur der Unbehaglichkeitsschwelle gemäß Tabelle 10 vorgenommen werden. Im genannten Beispiel empfindet der Proband z.B. das Signal als schmerzhaft laut. Die Unbehaglichkeitsschwellen der Tabelle 9 werden dementsprechend ausgehend von den in Tabelle 10 ablesbaren Werten um 5 dBHL verringert. Bei 1 kHz ergibt sich dementsprechend eine individuelle Unbehaglichkeitsschwelle für den betrachteten Probanden von 95 dBHL.

Entsprechende weitere Abschätzungen des Hörverlustes werden für weitere verschiedene Frequenzwerte durchgeführt, so dass der Hörverlust im interessierenden Frequenzbereich bestimmt werden kann.

In einem abschließenden Schritt wird der Proband einer Auralisierung unterzogen. Mit einem handelsüblichen akustischen Wiedergabesystem werden ihm definierte Schalleigenschaften beidseitig hörbar gemacht, wobei die gemäß obiger Verfahrensführung bestimmte Abschätzung des Hörverlustes berücksichtigt wird, indem eine lineare Verstärkung aus den ermittelten Hörverlust mit dem Wiedergabesystem realisiert wird. Der Proband sollte dementsprechend bereits einen besseren Höreindruck haben.

Dem Probanden wird jetzt die Möglichkeit gegeben, die Lautstärke, die Klangeinstellung oder die Balance des vorgespielten Signals zu korrigieren, um sein subjektives Empfinden einfließen zu lassen.

Es hat sich gezeigt, dass durch die entsprechende Anwendung des erfindungsgemäßen Verfahrens eine Schätzung des Hörverlustes und entsprechende Einstellung der Hörgeräte mit einer Genauigkeit möglich ist, die die Anwendung eines kostenintensiven und wartungsintensiven Audiometers überflüssig macht.

## Patentansprüche

1. Verfahren zur automatisierten Bestimmung der individuellen Hörfähigkeit mit folgenden Schritten:

   a) automatische Berechnung des statistischen frequenzabhängigen Altershörverlustes HV als erste frequenzabhängige Basis für die Abschätzung der frequenzabhängigen Hörfähigkeit eines Probanden aus Alter und Geschlecht des Probanden und in einer Speichereinrichtung abgelegten geschlechtsabhängigen Parametern, und

   b) Korrektur der so berechneten frequenzabhängigen Basis für die Abschätzung des frequenzabhängigen Altershörverlustes durch Berücksichtigung der Ergebnisse einer strukturierten Befragung des Probanden in Form automatisch berechneter frequenzabhängiger Korrekturwerte für die Abschätzung des individuellen Hörverlustes.

2. Verfahren zur automatisierten Bestimmung der individuellen Hörfähigkeit nach Anspruch 1, **dadurch gekennzeichnet, dass** die automatische Berechnung des statistischen frequenzabhängigen Altershörverlustes HV nach der Formel

$$HV(f) = 1{,}57 \cdot a(f) \cdot (Y-18)^2 + 1{,}28 \cdot b(f)$$

erfolgt, wobei a und b Funktionen der Frequenz, Y das Alter des Probanden ist.

3. Verfahren zur automatisierten Bestimmung der individuellen Hörfähigkeit nach einem der Ansprüche 1 oder 2, **dadurch gekennzeichnet, dass** aus den Ergebnissen der strukturierten Befragung auf einen Hörschadenstyp bzw. auf Hörschadenstypen des Probanden geschlossen wird.

4. Verfahren zur automatisierten Bestimmung der individuellen Hörfähigkeit nach Anspruch 3, **dadurch gekennzeichnet, dass** zur Korrektur der frequenzabhängigen Basis für die Abschätzung des frequenzabhängigen Altershörverlustes automatisch Korrekturwerte, vorzugsweise frequenzabhängige Korrekturwerte, in Abhängigkeit des Hörschadenstyps bestimmt werden.

**5.** Verfahren zur automatisierten Bestimmung der individuellen Hörfähigkeit nach einem der Ansprüche 1 bis 4, **dadurch gekennzeichnet, dass** die frequenzabhängigen Werte für die Abschätzung des Hörverlustes zusätzlich durch Berücksichtigung der Sprachverständlichkeit korrigiert werden.

**6.** Verfahren zur automatisierten Bestimmung der individuellen Hörfähigkeit nach Anspruch 5, **dadurch gekennzeichnet, dass** die Sprachverständlichkeit durch eine Sprachverständlichkeitsbewertung für mehrere, vorzugsweise mindestens drei, Situationen für Sprachverstehen ermittelt wird.

**7.** Verfahren zur automatisierten Bestimmung der individuellen Hörfähigkeit nach einem der Ansprüche 5 oder 6, **dadurch gekennzeichnet, dass** die Sprachverständlichkeit in mehreren, vorzugsweise fünf, Stufen bestimmt wird.

**8.** Verfahren zur automatisierten Bestimmung der individuellen Hörfähigkeit nach einem der Ansprüche 5 bis 7, **dadurch gekennzeichnet, dass** die Korrekturwerte des Hörverlustes zur Berücksichtigung der Sprachverständlichkeit ein Produkt aus einer Sprachverständlichkeitsbewertung und ausgewählten Signalpegeln umfassen.

**9.** Verfahren zur automatisierten Bestimmung der individuellen Hörfähigkeit nach Anspruch 8, **dadurch gekennzeichnet, dass** die Korrekturwerte zur Berücksichtigung der Sprachverständlichkeit eine frequenzabhängige Wichtung, insbesondere zur Konzentration auf mittlere Frequenzen im hörbaren Bereich umfassen.

**10.** Verfahren zur automatisierten Bestimmung der individuellen Hörfähigkeit nach einem der Ansprüche 1 bis 9, **dadurch gekennzeichnet, dass** zusätzlich zum frequenzabhängigen Hörverlust die Unbehaglichkeitsschwelle des Probanden korrigiert wird.

**11.** Verfahren zur automatisierten Bestimmung der individuellen Hörfähigkeit nach einem der Ansprüche 1 bis 10, **dadurch gekennzeichnet, dass** die frequenzabhängigen Werte für die Abschätzung des Hörverlustes durch Berücksichtigung der seitenspezifischen Wahrnehmung des Probanden korrigiert werden.

**12.** Verfahren zur automatisierten Bestimmung der individuellen Hörfähigkeit nach Anspruch 11, **dadurch gekennzeichnet, dass** die seitenspezifische Wahrnehmung des Probanden aus den Ergebnissen der Befragung ermittelt wird.

**13.** Verfahren zur automatisierten Bestimmung der individuellen Hörfähigkeit nach einem der Ansprüche 1 bis 12, **dadurch gekennzeichnet, dass** die frequenzabhängigen Werte für die Abschätzung des Hörverlustes abschließend im Rahmen einer Auralisierung, vorzugsweise durch Einstellung von Lautstärke und/oder Balance und/oder Klang durch den Probanden an einem handelsüblichen akustischen Wiedergabesystem korrigiert werden.

**14.** Verfahren zur Hörgeräteanpassung, bei dem ein Verfahren nach einem der Ansprüche 1 bis 13 zur automatisierten Bestimmung der individuellen Hörfähigkeit eines Probanden eingesetzt wird, und die Einstellung des Hörgerätes bzw. der Hörgeräte des Probanden in Abhängigkeit der so bestimmten individuellen Hörfähigkeit vorgenommen wird.

**Claims**

**1.** A method for the automated determination of the individual hearing ability comprising the following steps:

a) automatic calculation of the statistical frequency-dependent age-related hearing loss HV as a first frequency-dependent basis for the estimate of the frequency-dependent hearing ability of a subject from the age and sex of the subject and from sex-dependent parameters stored in a memory device; and
b) correcting the thus calculated frequency-dependent basis for the estimate of the frequency-dependent age-related hearing loss by taking account of the results of a structured questioning of the subject in the form of automatically calculated frequency-dependent correction values for the estimate of the individual hearing loss.

**2.** A method for the automated determination of the individual hearing ability in accordance with claim 1, **characterised in that** the automatic calculation of the statistical frequency-dependent age-related hearing loss HV takes place according to the formula

$$HV(f) = 1.57 \cdot a(f) \cdot (Y-18)^2 + 1.28 \cdot b(f)$$

where a and b are functions of the frequency and Y is the age of the subject.

**3.** A method for the automated determination of the individual hearing ability in accordance with one of the claims 1 or 2, **characterised in that** a conclusion is drawn on a type of hearing defect or of on types of hearing defect of the subject from the results of the structured questioning.

**4.** A method for the automated determination of the individual hearing ability in accordance with claim 3, **characterised in that** automatic correction values, preferably frequency-dependent correction values, are determined in dependence on the type of hearing defect for correcting the frequency-dependent basis for the estimate of the frequency-dependent age-related hearing loss.

**5.** A method for the automated determination of the individual hearing ability in accordance with any one of the claims 1 to 4, **characterised in that** the frequency-dependent values for the estimate of the hearing loss are additionally corrected by taking account of the speech intelligibility.

**6.** A method for the automated determination of the individual hearing ability in accordance with claim 5, **characterised in that** the speech intelligibility is determined by a speech intelligibility assessment for a plurality of situations, preferably at least three situations, for speech comprehension.

**7.** A method for the automated determination of the individual hearing ability in accordance with one of the claims 5 or 6, **characterised in that** the speech intelligibility is determined in a plurality of steps, preferably five steps.

**8.** A method for the automated determination of the individual hearing ability in accordance with any one of the claims 5 to 7, **characterised in that** the correction values of the hearing loss for taking account of the speech intelligibility include a product from a speech intelligibility assessment and from selected signal levels.

**9.** A method for the automated determination of the individual hearing ability in accordance with claim 8, **characterised in that** the correction values for taking account of the speech intelligibility include a frequency-dependent weighting, in particular for concentrating on middle frequencies in the audible spectrum.

**10.** A method for the automated determination of the individual hearing ability in accordance with any one of the claims 1 to 9, **characterised in that** the level of discomfort of the subject is corrected in addition to the frequency-dependent hearing loss.

**11.** A method for the automated determination of the individual hearing ability in accordance with any one of the claims 1 to 10, **characterised in that** the frequency-dependent values for the estimate of the hearing loss are corrected by taking account of the side-specific perception of the subject.

**12.** A method for the automated determination of the individual hearing ability in accordance with claim 11, **characterised in that** the side-specific perception of the subject is determined from the results of the questioning.

**13.** A method for the automated determination of the individual hearing ability in accordance with any one of the claims 1 to 12, **characterised in that** the frequency-dependent values for the estimate of the hearing loss are finally corrected at a commercial acoustic reproduction system within the framework of an auralisation, preferably by setting the volume and/or balance and/or tone by the subject.

**14.** A method for fitting hearing aids, wherein a method in accordance with any one of the claims 1 to 13 is used for the automated determination of the individual hearing ability of a subject and the setting of the hearing aid or of the hearing aids by the subject is carried out in dependence on the thus determined individual hearing ability.

**Revendications**

**1.** Procédé pour la détermination automatisée de la capacité auditive individuelle, comprenant les étapes suivantes :

a) calcul automatique de la perte auditive statistique en dépendance de la fréquence sous l'effet de l'âge (HV) à titre de première base dépendante de la fréquence pour l'estimation de la capacité auditive dépendante de la fréquence d'un sujet testé à partir de l'âge et du sexe du sujet testé et à partir de paramètres dépendants du sexe déposés dans un dispositif à mémoire, et

b) correction de la base dépendante de la fréquence ainsi calculée pour l'estimation de la perte auditive en dépendance de la fréquence sous l'effet de l'âge par prise en compte des résultats d'une interrogation structurée du sujet testé sous la forme de valeurs de correction dépendantes de la fréquence et calculées automatiquement pour l'estimation de la perte auditive individuelle.

2. Procédé pour la détermination automatisée de la capacité auditive individuelle selon la revendication 1, **caractérisé en ce que** le calcul automatique de la perte auditive statistique dépendante de la fréquence sous l'effet de l'âge (HV) a lieu d'après la formule

$$HV(f) = 1{,}57 \cdot a(f) \cdot (Y\ \text{-}18)^2 + 1{,}28 \cdot b(f)$$

dans laquelle a et b sont des fonctions de la fréquence et Y est l'âge du sujet testé.

3. Procédé pour la détermination automatisée de la capacité auditive individuelle selon l'une des revendications 1 ou 2, **caractérisé en ce qu'**à partir des résultats de l'interrogation structurée on procède à une conclusion quant à un type de dommage auditif ou à des types de dommages auditifs du sujet testé.

4. Procédé pour la détermination automatisée de la capacité auditive individuelle selon la revendication 3, **caractérisé en ce que** pour la correction de la base dépendante de la fréquence pour l'estimation de la perte auditive dépendante de la fréquence sous l'effet de l'âge, on détermine automatiquement des valeurs de correction, de préférence des valeurs de correction dépendantes de la fréquence, en fonction du type de dommage auditif.

5. Procédé pour la détermination automatisée de la capacité auditive individuelle selon l'une des revendications 1 à 4, **caractérisé en ce que** les valeurs dépendantes de la fréquence pour l'estimation de la perte auditive sont additionnellement corrigées en prenant en compte la compréhensibilité linguistique.

6. Procédé pour la détermination automatisée de la capacité auditive individuelle selon la revendication 5, **caractérisé en ce que** la compréhensibilité linguistique est déterminée par une évaluation de compréhensibilité linguistique pour plusieurs situations, de préférence au moins trois, pour la compréhension linguistique.

7. Procédé pour la détermination automatisée de la capacité auditive individuelle selon l'une des revendications 5 ou 6, **caractérisé en ce que** la compréhensibilité linguistique est déterminée en plusieurs niveaux, de préférence cinq.

8. Procédé pour la détermination automatisée de la capacité auditive individuelle selon l'une des revendications 5 à 7, **caractérisé en ce que** les valeurs de correction de la perte auditive pour la prise en compte de la compréhensibilité linguistique incluent un produit d'une évaluation de compréhensibilité linguistique et de niveaux de signaux choisis.

9. Procédé pour la détermination automatisée de la capacité auditive individuelle selon la revendication 8, **caractérisé en ce que** les valeurs de correction pour la prise en compte de la compréhensibilité linguistique comprennent une pondération dépendante de la fréquence, en particulier pour la concentration à des fréquences moyennes dans la plage audible.

10. Procédé pour la détermination automatisée de la capacité auditive individuelle selon l'une des revendications 1 à 9, **caractérisé en ce que**, additionnellement à la perte auditive dépendante de la fréquence, on corrige le seuil de gêne du sujet testé.

11. Procédé pour la détermination automatisée de la capacité auditive individuelle selon l'une des revendications 1 à 10, **caractérisé en ce que** les valeurs dépendantes de la fréquence pour l'estimation de la perte auditive sont corrigées par prise en compte de la perception spécifique latérale du sujet testé.

12. Procédé pour la détermination automatisée de la capacité auditive individuelle selon la revendication 11, **caractérisé en ce que** la perception spécifique latérale du sujet testé est déterminée à partir des résultats de l'interrogation.

EP 1 933 591 B1

**13.** Procédé pour la détermination automatisée de la capacité auditive individuelle selon l'une des revendications 1 à 12, **caractérisé en ce que** les valeurs dépendantes de la fréquence pour l'estimation de la perte auditive sont ensuite corrigées dans le cadre d'une auralisation, de préférence par réglage du niveau sonore et/ou de l'équilibrage et/ou de la sonorité par le sujet testé sur un système de restitution acoustique habituel du commerce.

**14.** Procédé pour l'adaptation d'un appareil auditif, dans lequel on emploie un procédé selon l'une des revendications 1 à 13 pour la détermination automatisée de la capacité auditive individuelle d'un sujet testé, et le réglage de l'appareil auditif ou des appareils auditifs du sujet testé est exécuté en dépendance de la capacité auditive individuelle ainsi déterminée.

Figur 2

Figur 1

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 1239269 A

**In der Beschreibung aufgeführte Nicht-Patentliteratur**

- **DIEROFF H.-G.** Lärmschwerhörigkeit. Gustav Fischer Verlag, 1994